# EUROPEAN PATENT APPLICATION

(11) **EP 2 095 836 A1**
(43) Date of publication of application: **02.09.2009**
(21) Application number: 07849846.6
(22) Date of filing: 17.12.2007
(51) Int. Cl.: A61M 5/28, A61J 1/05

(54) **SYRINGE OUTER TUBE FOR CHEMICAL SOLUTION FILLED AND SEALED SYRINGE FORMULATION AND PROCESS FOR PRODUCING THE SAME**

(30) Priority: 18.12.2006 JP 2006339438
(71) Applicant: Daiichi Sankyo Company, Limited, Chuo-ku Tokyo 103-8426 (JP)
(72) Inventor: NAGATA, Ryoh, Hiratsuka-shi Kanagawa 254-0014 (JP); ANDO, Yukihiro, Hiratsuka-shi Kanagawa 254-0014 (JP); SAITO, Morisaku, Tokyo 116-0002 (JP)
(74) Representative: Blodig, Wolfgang
(86) International application number: PCT/JP2007/001416
(87) International publication number: WO 2008/075460

(57) **Abstract**

A syringe barrel for pre-filled syringe formulation capable of suppressing the adhesion of air bubbles, and a pre-filled syringe formulation having the adhesion of airbubbles suppressed, are provided, and a process allowing efficient production of the syringe barrel is provided.

A syringe barrel for pre-filled syringe formulation having the interior surface treated by corona discharge; a pre-filled syringe formulation having a drug solution filled and sealed in the syringe barrel; and a process for producing a syringe barrel for pre-filled syringe formulation, including subjecting the interior surface of a syringe barrel to a corona discharge treatment.

## Description

### TECHNICAL FIELD

The present invention relates to a syringe barrel to be used for a syringe formulation which has preliminarily been filled with drug solution and sealed it in an injection barrel (hereinafter referred to as "a pre-filled syringe formulation"), a process for producing the barrel, and a syringe formulation which has been filled with a drug solution and sealed it (i.e. "pre-filled syringe formulation").

### BACKGROUND ART

Of the conventional syringe barrels for pre-filled syringe formulations, a specific one made of synthetic resins is widely used because of its simplicity of use.
However, such conventional syringe barrels are problematic in that air bubbles tend to easily adhere to their interior surfaces. This could lead to a serious consequence (e.g. air embolism), if such air bubbles actually intrude into the blood vessels during the administering of a drug solution. For this reason, whenever the administration of a drug solution is performed by use of a conventional pre-filled syringe formulation, a physician has been required to take extra care, lest air bubbles intrude into the blood vessels. For example, a physician used to rely up on a method by which air bubbles adhering to the inner can be accumulated into one area by striking the syringe tube with fingers in advance. However, such a method has another problem, because an extreme delay would be invited on the treatment or checkup of patients, if this method is taken at a busy clinical scene.

So far, there has been a report on a technologywhich enables the adhesion of air bubbles to be reduced in order to prevent the above-mentioned problems before they occur, by subjecting the interior surface of the syringe tube to an oxygen plasma treatment to increase hydrophilicity (e.g. Patent Document 1).
However, this oxygen plasma treatment requires massive amounts of equipments and also is not suitable for continuous treatment in terms of the production line, so that the realization of efficient production stays difficult as it now stands.
Patent Document 1: JP 2004-534596 A

### DISCLOSURE OF THE INVENTION

### Problems to be Solved by the Invention

The present invention was made in light of such longstanding problems and circumstances as described above, and it is an object of the invention to provide a syringe barrel for pre-filled syringe formulation, which is capable of suppressing the adhesion of air bubbles to the interior surface, apre-filled syringe which is capable of suppressing the adhesion of air bubbles, and a process for efficiently producing the syringe barrel.

### Means for Solving the Problems

The inventors of the present invention conducted various studies intended to solve the problems described above, and as a result, they found that when the interior surface of a syringe barrel is treated by corona discharge, the adhesion of air bubbles to the interior surface is significantly suppressed. Thus the present invention was completed.

The present invention has solved the above-described problems by means of a syringe barrel for drag solution filled and sealed syringe formulation, which is characterized by having the interior surface treated by corona discharge.

The present invention has also solved the above-described problems by means of a pre-filled syringe formulation, which is characterized by having a drug solution filled and sealed in the aforementioned syringe barrel.

The present invention has also solved the above-described problems by means of a process for producing a syringe barrel for pre-filled syringe formulation, which is characterized by having a process of subj ecting the interior surface of the syringe barrel to a corona discharge treatment.

### Advantageous Effects of the Invention

By use of the syringe barrel of the present invention, a pre-filled syringe formulation capable of suppressing the adhesion of air bubbles to the interior surface can be provided.
Furthermore, since the adhesion of air bubbles to the interior surface can be suppressed by use of the pre-filled syringe formulation of the present invention, there is no need to perform an operation of collecting the air bubbles adhering to the interior surface of the syringe barrel to one area when a physician injects a drug solution at a clinical scene, and thus administration of drug solution can be performed more efficiently and safely.
Moreover, according to the present invention, since massive amounts of equipments are not required, and continuous treatment is facilitated, highly efficientproductionof syringe barrels which are capable of suppressing the adhesion of air bubbles is made possible.

### BRIEF DESCRIPTION OF THE DRAWINGS

Fig. 1 is an explanatory outline cross-sectional view of a pre-filled syringe formulation using the syringe barrel of the present invention.
Fig. 2a is a schematic explanatory diagram showing the contact angle at the surface of a syringe barrel which has not been subjected to corona discharge treatment.
Fig. 2b is a schematic explanatory diagram showing the contact angle at the surface of a syringe barrel which has been subjected to corona discharge treatment.
Fig. 3 is a diagram showing the results of the state of air bubble adhesion in the corona discharge treated syringe barrel used in Test Example 2.
Fig. 4 is a diagram showing the results of the state of air bubble adhesion in the non-corona discharge treated syringe barrel used in Test Example 2.
Fig. 5 is a diagram showing the results of the state of air bubble adhesion in the corona discharge treated syringe barrel used in Test Example 3.
Fig. 6 is a diagram showing the results of the state of air bubble adhesion in the non-corona discharge treated syringe barrel used in Test Example 3.

### Reference Numerals

- 10:: Pre-filled syringe formulation
- 11:: Syringe barrel
- 12:: Cap
- 13:: Plunger
- P:: Drug solution
- θ:: Contact angle

### BEST MODE FOR CARRYING OUT THE INVENTION

Hereinafter, embodiments of the present invention will be described with reference to the drawings.

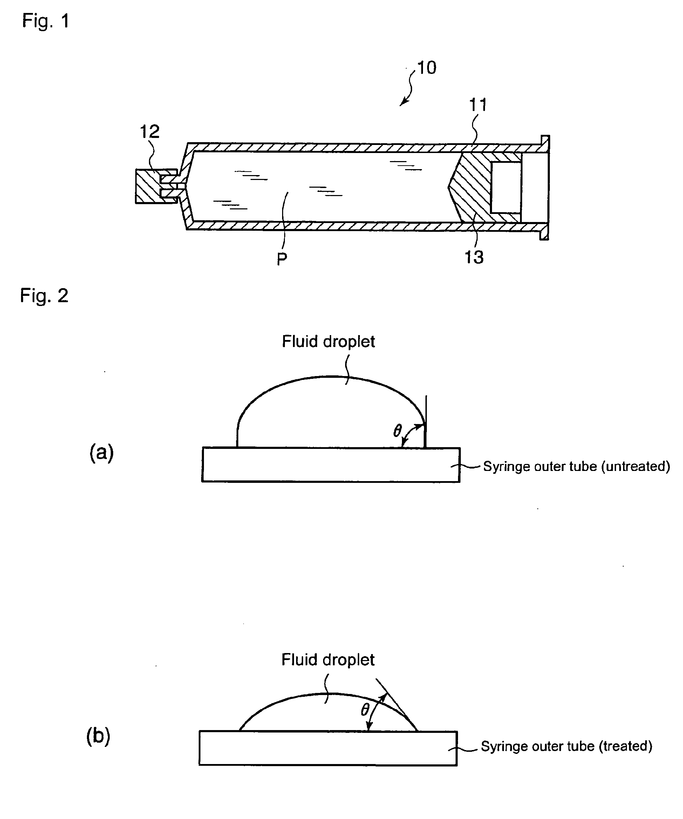

Fig. 1 is an explanatory outline cross-sectional view of a pre-filled syringe formulation using the syringe barrel of the present invention.
In Fig. 1, reference numeral 10 represents a pre-filled syringe formulation. A syringe barrel 11 is filled in the inside with a drug solution P, and the tip nozzle part thereof is sealed by a freely attachable and detachable cap 12, while the opening of the tail part thereof is sealed by a freely sliding plunger 13 at the same time.

The interior surface of the syringe barrel 11 has been subjected to a corona discharge treatment, so that a contact angle θ of 50 to 80° is obtained for a fluid droplet on the surface (see Fig. 2(b)).
As for the condition of corona discharge treatment resulting in such a contact angle θ, it is desirable to carry out the treatment with a corona discharge treatment apparatus, for which an output power and a duration of treatment sufficient for obtaining the aforementioned effect have been appropriately set in accordance with the size or shape of the syringe barrel 11, but for example, in the case of a syringe barrel for contrast agents, it is preferable to carry out the treatment at an output power of 50 to 400 W for about 5 to 10 seconds.
Furthermore, the corona discharge treatment apparatus is not particularly limited, but for example, corona discharge treatment apparatuses manufactured by Kasuga Electric Works, Ltd. are suitably used.

The material of the syringe barrel 11 is not particularly limited, but the material is preferably a synthetic resin. Examples of such synthetic resin include polypropylene resins, polyethylene resins, cyclic polyolefin resins and the like, but a product molded from a cyclic polyolefin resin is preferred from the viewpoint that the resin has excellent transparency, and it can be confirmed by visual inspection as to whether generation of air bubbles is suppressed. As for the cyclic polyolefin resin, there maybe mentioned, for example, synthetic resins "trade name: ZEONEX (registered trademark) " manufactured by Zeon Corporation; synthetic resins "trade name: APEL (registered trademark) "manufactured by Mitsui Chemicals, Inc.; synthetic resins "trade name: TOPAS (registered trademark)" manufactured by Topas Advanced Polymers GmbH; and the like.

The drug solution P is a solution or suspension of a drug which is applicable as the pre-filled syringe formulation, and is not particularly limited as long as it is an injectable solution prepared for injection. For example, an injectable solution such as a contrast agent, a cardiovascular agent, an antispasmodic , a local anesthetic drug, an infusion formulation, a vitamin formulation, a vaccine for prophylactic inoculation, an antiplasmin agent, or a hyaluronan formulation can be used.
Here, examples of the contrast agent include injectable solutions of nonionic iodinated X-ray contrast agents such as iopromide, iomeprol, iopamidol, ioversol, iohexol, ioxilan, iotrolan and iodixanol; ionic iodinated X-ray contrast agents such as sodium meglumine amidotrizoate, sodium iolactamate, meglumine iolactamate, ioxaglic acid and meglumine iotroxate; nonionic MRI contrast agents such as gadodiamide hydrate and gadoteridol; ionic MRI contrast agents such as meglumine gadoterate and meglumine gadopentetate; and the like.
Examples of the cardiovascular agent include injectable solutions of anticoagulants such as heparin, heparin sodium and argatrovan; cardiotonic agents such as epinephrine and norepinephrine; and the like.
Examples of the antispasmodic include injectable solutions of butylscopolamine bromide, and the like.
Examples of the local anesthetic drug include injectable solutions of lidocaine hydrochloride, dibucaine hydrochloride, bupivacaine hydrochloride, lopivacaine hydrochloride, and the like.
Examples of the infusion formulation include injectable solutions of calcium chloride, glucose, sodium chloride, magnesium sulfate, dipotassium phosphate, and the like.
Examples of the vitamin formulation include injectable solutions of vitamin A, vitamin B₁, vitamin B₂, vitamin B₆, vitamin B₁₂, vitamin C, vitamin D, vitamin E, calcium pantothenate, nicotinic acid amide, retinol palmitate, mixed vitamins combining these, and the like.
Examples of the vaccine for prophylactic inoculation include injectable solutions of influenza HA vaccine, adsorbed diphtheria-purified pertussis-tetanus combined vaccine (DPT), rubella vaccine, mumps vaccine, varicella vaccine, hepatitis B vaccine, and the like.
Examples of the antiplasmin agent include injectable solutions of tranexamic acid and the like.
According to the present invention, among the drug solutions as described above, the injectable solutions of nonionic iodinated X-ray contrast agents, inter alia, the injectable solution of iohexol and the injectable solution of iodixanol are particularly preferred.

### Examples

Hereinafter, the present invention will be described in more detail by way of Examples.

### Example 1

The entire interior surface of a syringe barrel made of a cyclic polyolefin resin (capacity 100 mL) was treated by means of a corona discharge treatment apparatus manufactured by Kasuga Electric Works, Ltd. at an output power of 250 W for about 5 seconds, and thus a syringe barrel for pre-filled syringe formulation of the present invention was obtained.

### Test Example 1

For each of the syringe barrel of the present invention obtained in Example 1 and a non-corona discharge treated syringe barrel made of a cyclic polyolefin resin, the contact angle θ of a fluid droplet formed on the surface (see Figs. 2(a) and 2(b)) was measured. As for the fluid droplet, an injectable solution of iohexol ("OMNIPAQUE: trade name" manufactured by Daiichi Sankyo Co. , Ltd.) and an injectable solution of iodixanol ("VISIPAQUE: trade name" manufactured by Daiichi Sankyo Co., Ltd.) were used, and the respective contact angles were measured.
The results are presented in Table 1.

**[Table 1]**

| | Fluid droplet | Syringe barrel of present invention | Untreated syringe barrel |
|---|---|---|---|
| Contact angle θ | Injectable solution of iohexol | 70° | 90° |
| | Injectable solution of iodixanol | 70° | 90° |

### Test Example 2

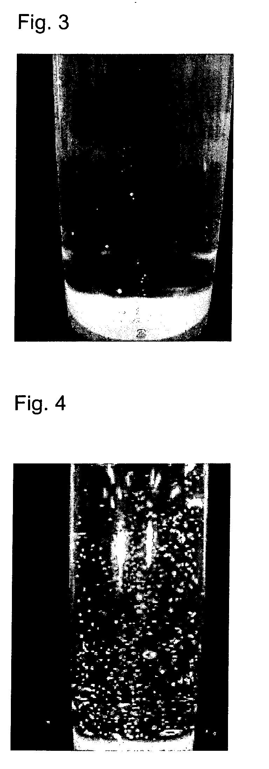

In each of the syringe barrel of the present invention obtained in Example 1 and the non-corona discharge treated syringe barrel made of a cyclic polyolefin resin, an injectable solution of iohexol ("OMNIPAQUE: trade name" manufactured by Daiichi Sankyo Co., Ltd.), which is a contrast agent, was filled and sealed. Air bubbles were completely removed in advance, and then an impact was applied to confirm the state of adhesion of air bubbles.
As a result, air bubbles were nearly not recognized in the syringe barrel of the present invention (Fig. 3), while a large number of air bubbles were recognized in the untreated syringe barrel (Fig. 4).

### Test Example 3

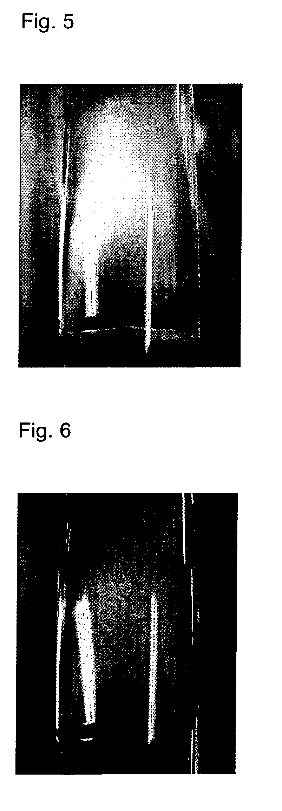

The injectable solution of iohexol according to Test Example 2 was changed to an injectable solution of iodixanol ("VISIPAQUE: trade name") manufactured by Daiichi Sankyo Co., Ltd.), and the state of adhesion of air bubbles was confirmed in the same manner as in Test Example 2.
As a result, air bubbles were hardly recognized in the syringe barrel of the present invention (Fig. 5), while a large number of air bubbles were recognized in the untreated syringe barrel (Fig. 6).

## Claims

1. A syringe barrel for pre-filled syringe formulation, comprising an interior surface treated by corona discharge.

2. The syringe barrel for pre-filled syringe formulation according to claim 1, wherein the syringe barrel is made of a synthetic resin.

3. The syringe barrel for pre-filled syringe formulation according to claim 1 or 2, wherein the syringe barrel is made of a cyclic polyolefin resin.

4. The syringe barrel for pre-filled syringe formulation according to any one of claims 1 to 3, wherein the interior surface has a contact angle of 50 to 80° against a fluid droplet on the surface.

5. A pre-filled syringe formulation having a drug solution filled and sealed in the syringe barrel according to any one of claims 1 to 4.

6. The pre-filled syringe formulation according to claim 5, wherein the drug solution is an injectable solution of a contrast agent.

7. The pre-filled syringe formulation according to claim 5 or 6, wherein the drug solution is an injectable solution of iohexol.

8. The pre-filled syringe formulation according to claim 5 or 6, wherein the drug solution is an injectable solution of iodixanol.

9. The pre-filled syringe formulation according to any one of claims 5 to 8, wherein the drug solution is sealed by a cap and a plunger.

10. A process for producing a syringe barrel for pre-filled syringe formulation, the process comprising subjecting the interior surface of the syringe barrel to a corona discharge treatment.

11. The process for producing a syringe barrel for pre-filled syringe formulation according to claim 10, wherein the interior surface is subj ected to a corona discharge treatment so that the contact angle thereof can become 50 to 80° against a fluid droplet on the surface.

12. The process for producing a syringe barrel for pre-filled syringe formulation according to claim 10 or 11, wherein the corona discharge treatment is performed at an output power of 50 to 400 W.

13. The process for producing a syringe barrel for pre-filled syringe formulation according to any one of claims 10 to 12, wherein the syringe barrel is made of a cyclic polyolefin resin.

14. A process for producing a syringe barrel for pre-filled syringe formulation, the process comprising subjecting a syringe barrel made of a cyclic polyolefin resin to a corona discharge treatment at an output power of 50 to 400 W, to thereby form an interior surface capable of generating a contact angle of 50 to 80° for a fluid droplet.
